# FASCICULE DE BREVET EUROPEEN

(11) **EP 0 813 851 B1**
(45) Date de publication et mention de la délivrance du brevet: **19.12.2001**
(21) Numéro de dépôt: 97490019.3
(22) Date de dépôt: 16.06.1997
(51) Int. Cl.: A61F 13/62

(54) **Article d'hygiène jetable comportant un système de fermeture à ruban intercalaire séparable**
Hygienischer Wegwerfartikel mit trennbarem Zwischenbandverschluss
Disposable hygiene article with a fastening system provided with a separable interlayer tape

(30) Priorité: 19.06.1996 FR 9607893
(43) Date de publication de la demande: 29.12.1997
(73) Titulaire: Proteco, 59494 Petite Forêt (FR)
(72) Inventeur: Brutin, Jean-Marc, 59780 Camphin en Pevele (FR)
(74) Mandataire: Hennion, Jean-Claude

(56) Documents cités:
- EP-A- 0 491 347
- WO-A-92/04001
- WO-A-95/25496
- FR-A- 2 087 805
- GB-A- 2 233 876

## Description

La présente invention concerne le domaine des article d'hygiène jetables tels que des couches-culottes dont la fixation sur l'utilisateur est réalisée à l'aide d'un système de fermeture du type mécanique.

De manière conventionnelle, les articles d'hygiène jetables, tels que couches-culottes, étaient fermés grâce à des systèmes d'attaches à rubans adhésifs qui sont fixés de part et d'autre de la partie arrière de l'article et qui sont appliqués, après positionnement de l'article sur l'utilisateur, sur la face extérieure de la partie avant de l'article.

Un tel système d'attaches adhésives comprend une partie fixe qui est située, lors de la production de l'article, latéralement de part et d'autre de la partie arrière de l'article et une patte d'attache qui déborde de l'article et qui est revêtue sur une face d'une enduction adhésive. Avant usage, la patte d'attache est repliée sur la partie fixe de telle sorte que l'enduction adhésive soit en contact avec une face non adhérente de la partie fixe. Lors de l'utilisation, on détache la patte d'attache de la partie fixe et on applique la face de la patte d'attache revêtue de l'enduction adhésive sur la partie avant de l'article.

On a déjà proposé par le document FR 2 516 757 de disposer sur la partie avant de l'article une bande de renforcement lisse ou embossée sur laquelle est appliquée la face revêtue de l'enduction adhésive de la patte d'attache. Le rôle de cette bande de renforcement est de permettre d'ouvrir l'article en retirant la patte d'attache, sans occasionner de déchirure de la partie avant de l'article, puis de refermer l'article en refixant la patte d'attache sur ladite bande de renforcement. Cette opération d'ouverture et de refermeture de l'article peut théoriquement être réalisée à plusieurs reprises.

En pratique il a été constaté que cette possibilité de fixation et refixation de la patte d'attache était limitée du fait des souillures possibles de l'enduction adhésive, souillures pouvant provenir des matières grasses ou des poudres mises en oeuvre par l'utilisateur. L'enduction adhésive ainsi souillée perd en partie sa capacité d'adhérence sur la bande de renforcement de sorte que la fermeture latérale de l'article n'est plus réalisée avec une force suffisante.

On a cherché à résoudre cette difficulté en proposant des systèmes d'attaches mécaniques, c'est à dire des systèmes dont les deux faces en contact présentent des éléments mécaniques de fixation, aptes à coopérer l'un avec l'autre. Il s'agit notamment de boucles pour une face et de crochets pour l'autre face, les crochets pénétrant dans les boucles. Ce type d'attache mécanique est notamment connu sous la dénomination VELCRO.

L'utilisation de ce type d'attache pour la fermeture d'articles d'hygiène jetables, du type couches-culottes, a déjà été proposée notamment dans le document EP 235 014.

Par ailleurs, on a cherché à proposer des articles d'hygiène jetables qui puissent être repliés et fermés sur eux-mêmes après usage grâce au système d'attaches adhésives existant. En d'autres termes, l'utilisateur, après avoir retiré l'article souillé, le replie ou l'enroule sur lui-même et met en oeuvre le système d'attaches adhésives existant pour assurer la fermeture après usage de l'article de telle sorte qu'il puisse être jeté sans risquer qu'il ne s'ouvre et que les souillures ne s'en échappent.

Ceci est rendu possible lorsque le système d'attache est du type adhésif, avec bande de renforcement permettant la refixation périodique grâce à une bande de renforcement qui n'altère pas définitivement le caractère adhésif de l'attache. Ainsi après usage c'est la même enduction adhésive qui est appliquée sur la face extérieure de la couche après repliement ou enroulement sur elle-même.

On a déjà cherché par le document EP 321 232 à proposer un article d'hygiène jetable qui comporte un système de fermeture combinant des propriétés adhésives et mécaniques. Plus précisément la patte d'attache comporte de manière juxtaposée une ou des zones comprenant des éléments d'attache mécanique et une ou des zones comprenant une enduction adhésive. Cette patte d'attache est appliquée sur la face extérieure de la partie avant de l'article qui est munie d'une bande de renforcement avec des éléments d'attaches mécaniques. Ainsi lors de la fermeture de l'article les deux types d'éléments fonctionnent simultanément, à savoir d'une part les éléments d'attaches mécaniques coopèrent les uns avec les autres de manière à réaliser la fixation mécanique de la patte et l'enduction adhésive adhère sur les éléments mécaniques sur lesquelles elle est appliquée réalisant une fixation adhésive. C'est normalement la fixation mécanique qui est prioritaire dans la fermeture normale de l'article. Lorsqu'il s'agit de réaliser la fermeture après usage, c'est au contraire la zone présentant une enduction adhésive qui est mise en oeuvre par application sur la face extérieure de l'article dans une zone qui ne comporte pas de bande de renforcement mécanique. Ainsi selon ce document EP 321 232 il est possible d'obtenir une fermeture très fiable de l'article avec réouverture et refermeture possibles à de multiples reprises, puis une fermeture également très fiable de l'article après usage grâce au système combiné de fermeture à propriétés mécaniques et adhésives.

Cependant selon le demandeur, la fiabilité de ce système de fermeture ne peut pas être véritablement assurée du fait que la zone à propriétés adhésives est mise en oeuvre à chaque opération de réouverture et refermeture, venant s'appliquer sur une partie de la bande de renforcement équipée d'éléments d'attaches mécaniques. Il peut donc également se produire des phénomènes de souillures de cette partie adhésive par des matières grasses ou des poudres mises en oeuvre par l'utilisateur lors de ces opérations d'ouverture et de fermeture.

Le but que s'est fixé le demandeur est de proposer un article d'hygiène jetable qui pallie l'inconvénient précité en présentant une fiabilité accrue des possibilités de fixation et refixation de la patte d'attache ainsi que de fermeture de l'article après usage.

Ce but est parfaitement atteint par l'article d'hygiène jetable de l'invention, du type couche-culotte, qui de manière connue comporte une feuille extérieure imperméable, une feuille intérieure perméable, un matelas absorbant pris en sandwich entre les deux dites feuilles, ledit article étant configuré avec une zone d'entrejambe, une partie avant et une partie arrière et comportant des moyens d'attaches fixés latéralement à la partie arrière et aptes à réaliser la solidarisation au moins temporaire des parties arrière et avant après positionnement de l'article sur l'utilisateur par application sur une ou des bandes de renforcement fixées sur la partie avant de la feuille externe imperméable.

De manière caractéristique selon l'invention, chaque moyen d'attache comporte des premiers éléments d'attache mécanique, l'article est équipé d'un ruban intercalaire séparable comportant sur une face une enduction adhésive apte à solidariser ledit ruban intercalaire et ladite bande de renforcement pendant l'usage et sur l'autre face des seconds éléments d'attache mécanique, aptes à coopérer avec lesdits premiers éléments pendant l'usage, et le ruban intercalaire comporte une extrémité préhensile permettant son retrait de ladite bande de renforcement et l'application de sa face adhésive sur la feuille extérieure après usage.

Certes dans le document GB-A-2 233 876 on a déjà proposé une couche-culotte dont les pattes d'attache latérales sont pourvues d'éléments d'attache mécanique du type crochets et d'un ruban intercalaire ayant une face adhésive et l'autre face équipée d'éléments d'attache mécanique du type boucle, apte à coopérer avec les éléments du type crochets pendant l'usage. Cependant ce ruban intercalaire est, lors de la première manipulation par l'usager, collé de manière définitive sur la feuille extérieure. Il ne s'agit pas d'un ruban intercalaire séparable au sens de l'invention qui nécessite obligatoirement la possibilité de retirer le ruban intercalaire grâce à la coopération dudit ruban avec la bande de renforcement et à la présence de l'extrémité préhensile du ruban intercalaire.

Selon une première variante de réalisation, chaque moyen d'attache consiste en une patte d'attache dont une extrémité est fixée latéralement sur un bord de la partie arrière et qui comporte sur une face les premiers éléments d'attache mécanique.

De préférence dans ce cas, la face interne du bord de la partie arrière comporte une zone non-adhérente et, avant utilisation, la patte latérale d'attache est repliée, le ruban séparable étant disposé entre ladite zone non-adhérente et la patte d'attache en sorte que sa face adhésive soit en contact avec la zone non adhérente et sa face pourvue des seconds éléments mécaniques soit en contact avec les premiers éléments mécaniques de la patte d'attache.

Ainsi, de manière originale, le ruban intercalaire séparable qui est intégré dans la patte d'attache latérale, vient lors de la première utilisation adhérer sur la bande de renforcement. Par contre, lorsqu'il s'agit d'ouvrir de nouveau l'article puis de le refixer ce sont les éléments d'attache mécaniques qui sont mis en oeuvre, le ruban intercalaire séparable restant quant à lui fixé temporairement sur la bande de renforcement. Enfin lorsqu'il s'agit d'effectuer la dernière opération de fermeture après usage, l'utilisateur retire la patte d'attache en se saisissant du ruban intercalaire séparable en sorte que la face revêtue de l'enduction adhésive est de nouveau dégagée et peut être utilisée pour venir s'appliquer sur la face extérieure de l'article après que celui-ci ait été replié ou enroulé sur lui-même.

Ainsi l'enduction adhésive n'est utilisée qu'à deux reprises seulement : la première lors de la première fermeture de l'article et la seconde lors de la fermeture après usage. Il n'y a donc quasiment pas de possibilité de souillure lors des opérations de réouverture et refermeture successives.

Selon une seconde variante de réalisation, chaque moyen d'attache consiste en une bande qui est pourvue des premiers éléments d'attaches mécaniques et qui est fixée selon un bord de la face interne de la partie arrière. Il n'y a donc pas à proprement parler de patte latérale qui déborde du bord de la partie arrière et qui est susceptible d'être repliée, avant utilisation.

De préférence, dans ce cas, avant utilisation, la face adhésive du ruban intercalaire séparable est appliquée sur la bande de renforcement.

Lors des différentes opérations d'ouverture et refermeture, ce sont les éléments d'attache mécaniques du ruban intercalaire et de la bande d'attache qui coopèrent. Par contre lorsqu'il s'agit d'effectuer la dernière opération de fermeture après usage, l'utilisateur retire la partie arrière en se saisissant du ruban intercalaire séparable en sorte que la face de celui-ci, revêtue de l'enduction adhésive, soit dégagée et peut être utilisée pour venir s'appliquer sur la face extérieure de l'article après que celui-ci ait été replié ou enroulé sur lui-même. Dans cette seconde variante, l'enduction adhésive du ruban intercalaire n'est utilisée qu'à une seule reprise, à savoir la fermeture après usage. Toute souillure lors des opérations de réouverture et refermeture est donc impossible.

Avantageusement le bord selon lequel est fixée la bande d'attache mécanique est rigidifié, par exemple par un élément de rigidification disposé sur la face extérieure de la partie arrière.

Selon un procédé particulièrement avantageux de fabrication :
a. on déroule en continu une bande complexe composée de la superposition d'une première bande en matériau plastique dont une des faces dite face intérieure est adhésive, une seconde bande ayant une face adhésive et une face pourvue d'éléments d'attache mécanique, la face adhésive de la seconde bande étant appliquée sur la face extérieure de la première bande,
b. on découpe transversalement des tronçons de ladite branche complexe, et
c. on fixe chaque tronçon sur la face extérieure de la partie avant d'un article d'hygiène du type couche-culotte, en sorte d'obtenir la pose à la fois de la bande de renforcement et du ruban intercalaire séparable.

Lorsqu'il s'agit d'une bande de renforcement unique s'étendant transversalement sur la largeur de la partie avant, la bande complexe comprend deux secondes bandes séparées l'une de l'autre, chacune d'un côté de la première bande.

Lors de la mise en oeuvre du procédé précité, le ruban intercalaire a exactement la même largeur que la bande de renforcement.

De préférence, le ruban intercalaire séparable est un ruban textile ou non-tissé, présentant une surface en boucles ou fibreuse, et l'autre face revêtue d'une enduction adhésive.

La présente invention sera mieux comprise à la lecture de la description de l'exemple préféré de réalisation d'une couche-culotte jetable équipée d'un système de fermeture comportant un ruban intercalaire séparable, illustré par le dessin annexé dans lequel :
La figure 1 est une représentation en perspective d'une couche avec bande de renforcement et équipée de pattes d'attache latérales,
La figure 2 est une vue en coupe partielle de la couche de la figure 1 selon la ligne II.II,
Les figures 3 à 6 illustrent en coupe le système d'attache lors des différentes opérations,
La figure 7 est une représentation en perspective d'une couche avec bande de renforcement sur laquelle sont apposés deux rubans intercalaires,
Et les figures 8 et 9 sont des illustrations du mode de préparation d'une bande complexe comportant la superposition d'une bande de renforcement et de deux rubans intercalaires.

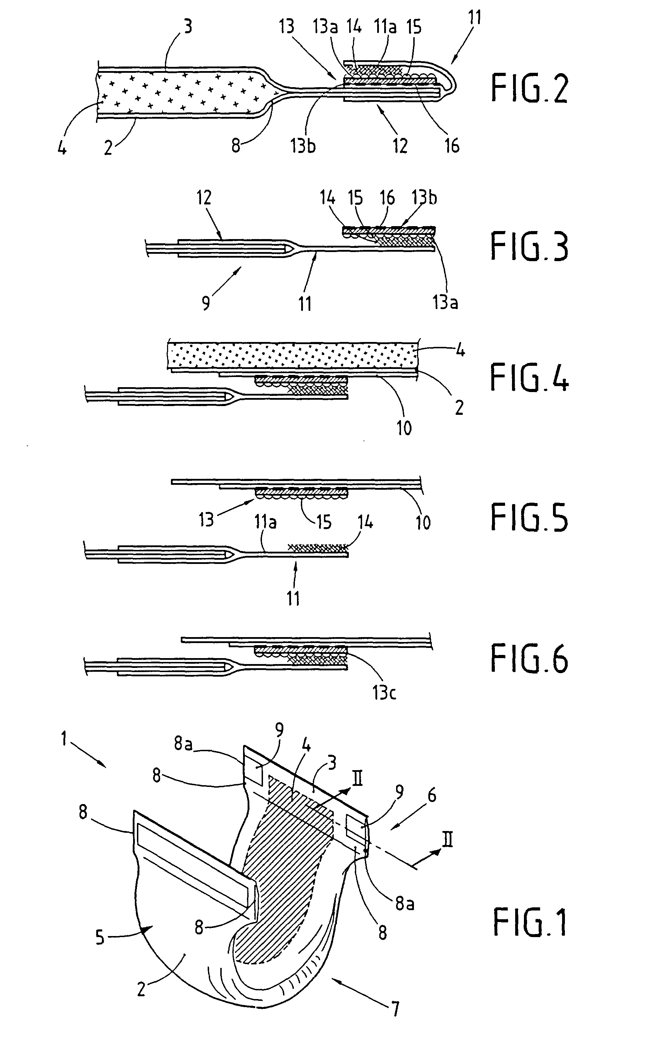

Dans un premier exemple de réalisation illustré aux figures 1 à 6, la couche-culotte 1 est un article d'hygiène jetable qui comprend une feuille extérieure imperméable 2, une feuille intérieure perméable 3 et un matelas absorbant 4 qui est pris en sandwich entre la feuille extérieure 2 et la feuille intérieure 3, lesdites feuilles étant solidarisées par exemple par soudage le long de leur contour périphérique. Dans l'exemple illustré à la figure 1, la couche culotte 1 comporte une partie avant 5 et une partie arrière 6 et une zone d'entrejambe 7. Les parties avant 5 et arrière 6 ont, dans l'exemple illustré, une largeur supérieure à celle d'entrejambe 7 de manière à former des rabats qui sont repliés les uns vers les autres lors de la fermeture de la couche 1 de sorte que la jambe de l'usager soit complètement entourée par la couche 1.

Selon les bords latéraux 8a des deux rabats 8 de la partie arrière 6 sont disposés des systèmes d'attache 9.

Sur la feuille extérieure imperméable 2 de la partie avant 5 est disposée transversalement une bande de renforcement 10 présentant une surface extérieure lisse conformément au document FR 2 516 757 ou embossée.

Le système d'attache 9, selon l'invention, tel qu'illustré à la figure 2, est constitué d'une patte d'attache 11 qui est fixée par une partie fixe 12 sur le rabat 8 et d'un ruban intercalaire séparable 13.

La patte d'attache 11 comporte sur une face 11a des éléments 14 d'attaches mécaniques, par exemple des éléments du type crochets ou griffes dans l'exemple illustré.

Le ruban intercalaire séparable 13 comporte sur une face 13a des éléments 15 d'attache mécaniques et sur l'autre face 13b une enduction adhésive 16. Les éléments d'attache mécaniques 15 du ruban intercalaire 13 sont complémentaires à ceux 14 de la patte d'attache 11 de sorte qu'ils coopèrent les uns avec les autres. Il présente une extrémité 13c préhensile, c'est-à-dire apte à pouvoir être saisie par l'utilisateur, comme cela sera expliqué ci-après. Cette extrémité 13c est par exemple exempte localement d'enduction adhésive 16.

Comme cela apparaît clairement à l'examen de la figure 2, lorsque la couche-culotte 1 est dans son emballage d'origine, avec son système d'attache 9 replié sur le rabat 8, les deux types d'éléments d'attache mécaniques 14 et 15 sont imbriqués l'un dans l'autre, tandis que l'enduction adhésive 16 portée par la face arrière 13b du ruban intercalaire 13 est en contact avec la partie fixe 12 du système d'attache 9, partie fixe qui recouvre le bord longitudinal du rabat 8. La surface de cette partie fixe 12 est à adhérence limitée de manière à ce que les forces d'accrochage entre les deux éléments d'attaches mécaniques 14,15 soient supérieures à la force de liaison entre l'enduction adhésive 16 et ladite surface de la partie fixe 12 du système d'attache 9. Ainsi lorsque l'utilisateur saisit l'extrémité libre du système d'attache, que ce soit l'extrémité proprement dite de la patte d'attache 11 ou l'extrémité libre du ruban intercalaire 13, la séparation se fait obligatoirement au niveau de l'interface entre l'enduction adhésive 16 et la partie fixe 12. Cette opération une fois réalisée est illustrée à la figure 3.

Dans l'exemple illustré aux figures 2 à 6, la patte d'attache 11 est fixée au rabat 8 par la partie fixe 12 qui comportent deux portions en Y prenant en sandwich le bord 8a du rabat 8. Ceci est un mode de réalisation parmi beaucoup d'autres. La patte 11 peut être soudée ou collée selon le bord 8a ou encore la partie fixe 11 peut elle-même être prise en sandwich entre la feuille extérieure imperméable 2 et la feuille intérieure perméable 3.

Lors de la mise en place de la couche-culotte 1 sur le bébé, après rapprochement des rabats 8 et ouverture du système d'attache 9 comme cela vient d'être décrit, l'utilisateur ferme latéralement la couche-culotte 1 en appliquant la patte d'attache 11 sur la bande de renforcement 10. Dans cette opération, c'est l'enduction adhésive 16 portée par le ruban intercalaire 13 qui vient en contact avec la surface lisse ou embossée de la bande de renforcement 10.

Lorsque l'utilisateur veut ouvrir de nouveau la couche-culotte 1, notamment pour vérifier si elle est ou non souillée, il utilise alors la patte d'attache 11, qu'il saisit à son extrémité libre 11a et sur laquelle il exerce une traction en sens inverse de telle sorte que les éléments 14 d'attaches mécaniques supportés par la patte d'attache 11 se désolidarisent des éléments 15 d'attaches mécaniques supportés par le ruban intercalaire 16. Ce faisant, le ruban intercalaire 13 reste fixé sur la bande de renforcement 10.

La refermeture de la couche-culotte 1, par refixation de la patte d'attache 11, est obtenue en appliquant celle-ci sur la surface extérieure du ruban intercalaire 13 de telle sorte qu'il y ait coopération entre les deux types 14 et 15 d'attache mécanique supportés respectivement par la patte d'attache 11 et le ruban intercalaire 13. Pour une nouvelle ouverture et fermeture de la couche-culotte, on procède comme cela vient d'être expliqué ci-dessus en mettant en oeuvre uniquement les éléments 14 et 15 d'attaches mécaniques.

Par contre lorsqu'il s'agit de jeter la couche-culotte 1, l'utilisateur doit se saisir de l'extrémité préhensile 13c du ruban intercalaire 13 et exercer une traction en sens inverse de sorte de désolidariser la bande de renforcement 10 et le ruban intercalaire au niveau de l'enduction adhésive 16, ruban intercalaire qui reste quant à lui fixé sur la patte d'attache 11 grâce à la coopération des deux types 14, 15 d'attaches mécaniques. On se retrouve alors dans la configuration qui est celle de la figure 3 c'est à dire la configuration qui existait lors de la première utilisation du système d'attache 9.

Après avoir replié ou enroulé sur elle-même la couche-culotte 1, l'utilisateur vient appliquer l'enduction adhésive 16 sur une zone quelconque de la feuille imperméable extérieure 2 de manière à obtenir la fermeture définitive après usage de la couche-culotte souillée.

Dans un exemple précis de réalisation; les éléments 14 d'attaches mécaniques qui étaient supportés par la patte d'attache 11 étaient des crochets ou des griffes, tandis que les éléments 15 d'attache mécaniques supportés par le ruban intercalaire 13 étaient des boucles ou fibres dégagées de la surface d'un matériau textile, par exemple par grattage. Cette disposition n'est pas exclusive, elle pourrait être inversée.

De même il n'est pas strictement nécessaire que la bande de renforcement 10 soit une bande unique disposée transversalement : ce pourrait être deux tronçons de bande disposés de part et d'autre au niveau des rabats latéraux 8.

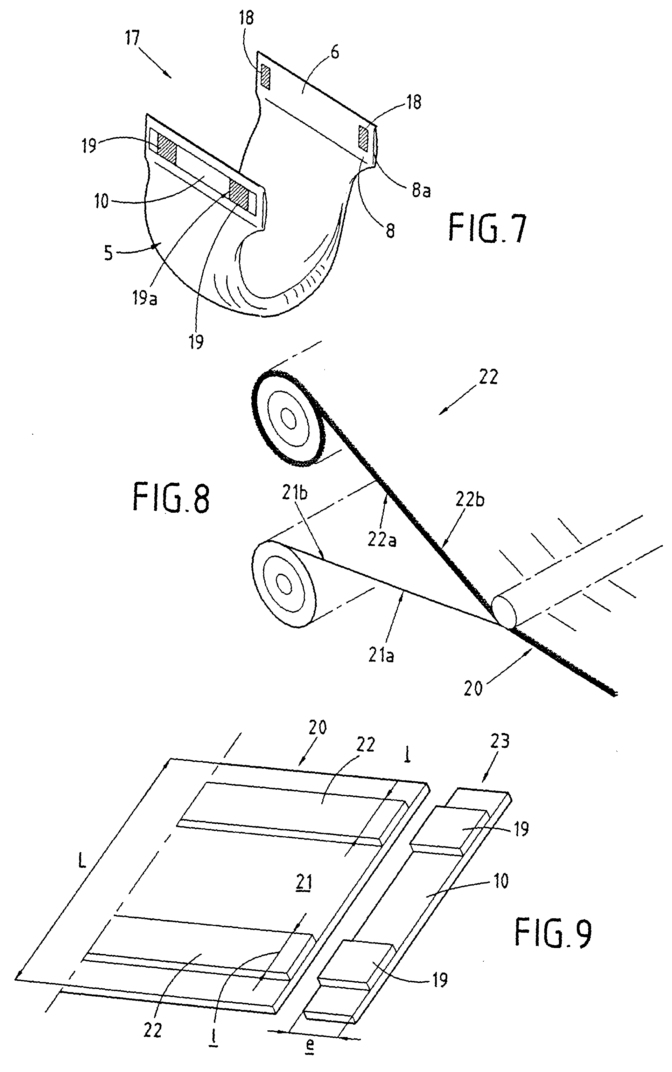

Dans le second exemple qui est illustré à la figure 7, la couche culotte 17 a la même structure générale que la couche culotte 1 du premier exemple, les éléments communs étant repris avec les mêmes références. De manière caractéristique, cette couche culotte 17 ne comporte pas à proprement parler de pattes d'attache qui débordent des rabats 8. Par contre sur la face intérieure des rabats 8 de la partie arrière sont disposées des bandes d'attache 18, à proximité des bords latéraux 8a desdits rabats 8. Ces bandes d'attache 18 sont des pièces rapportées, découpées dans une bande continue dont une face présente des éléments d'attache mécaniques, par exemple des éléments du type crochets ou griffes. Cette bande d'attache 18 est fixée notamment par collage sur la face intérieure de la partie avant à proximité du bord latéral 8a du rabat 8, comme illustré à la figure 7.

Sur la face extérieure de la partie avant 5 est disposée transversalement une bande de renforcement 10 à surface lisse ou embossée, comme dans le premier exemple. Cependant de manière caractéristique vers les extrémités de la bande de renforcement 10 sont disposés deux rubans intercalaires séparables 19 dont les faces comportant l'enduction adhésive sont appliquées sur la surface lisse ou embossée de la bande de renforcement 10.

Lorsque l'utilisateur prélève la couche 17 dans son emballage, celle-ci présente la configuration telle qu'illustrée à la figure 7.

Lors de la mise en place de la couche culotte 17 sur le bébé, après rapprochement des rabats 8, l'utilisateur ferme latéralement la couche culotte 17 en appliquant la bande d'attache 18 sur le ruban intercalaire 19 dont la face extérieure est celle qui comporte des éléments d'attache mécaniques destinés à coopérer avec les éléments d'attache mécaniques de la bande d'attache 18.

Lorsque l'utilisateur veut ouvrir de nouveau la couche culotte 17, il se saisit du bord latéral 8a du rabat 8 sur lequel il exerce une traction de telle sorte que la bande d'attache 18 supportée par ledit rabat 8 se désolidarise des éléments d'attache mécaniques supportés par le ruban intercalaire 19, celui-ci restant fixé sur la bande de renforcement 10.

Lorsqu'il s'agit de jeter la couche culotte 17, lors de l'ouverture de celle-ci l'utilisateur doit se saisir de l'extrémité préhensile 19a du ruban intercalaire 19, extrémité dont la face en contact avec la bande de renforcement 10 ne comporte pas d'enduction adhésive ; il exerce alors une traction en sens inverse en sorte de désolidariser la bande de renforcement 10 et le ruban intercalaire 19 au niveau de l'enduction adhésive supportée par ledit ruban intercalaire 19. Ce faisant le ruban intercalaire 19 reste fixé sur le rabat 8 grâce à la coopération des élément d'attache mécaniques d'une part de la bande d'attache 18 et d'autre part du ruban intercalaire 19.

Après avoir replié ou enroulé sur elle-même la couche culotte 17, l'utilisateur vient appliquer l'enduction adhésive supportée par le ruban intercalaire 19 sur une zone quelconque de la feuille imperméable extérieure 2 de manière à obtenir la fermeture définitive après usage de la couche culotte 17 souillée.

Les figures 8 et 9 illustrent un mode préféré de fabrication qui permet d'appliquer en une seule opération sur la partie avant 5 de la couche culotte à la fois la bande de renforcement 10 et les deux rubans intercalaires 19.

Selon ce procédé, on forme une bande complexe 20 qui est composée de la superposition d'une première bande 21 et d'une seconde bande 22. La première bande 21a une face 21a qui est recouverte d'une enduction adhésive et une face opposée 21b. La seconde bande 22 a une face 22a qui est recouverte d'une enduction adhésive et une face 22b qui est pourvue d'éléments d'attache mécaniques. La superposition des deux bandes 21 et 22 est réalisée de telle sorte que la face adhésivée 22a de la seconde bande 22 est appliquée sur la face opposée 21b de la première bande 21.

Plus précisément dans l'exemple illustré aux figures 7 à 9, la bande complexe 20 est composée d'une première bande continue 21 ayant une largeur L et de deux secondes bandes 22 de largeur nettement inférieure à L. Les deux secondes bandes 22 sont disposées parallèlement l'une à l'autre selon les bords opposés de la première bande 21 comme cela apparaît clairement à la figure 9.

On découpe transversalement dans ladite bande complexe 20 des tronçons 23. Chaque tronçon 23 est ensuite fixé sur la face extérieure de la partie avant 5 d'une couche culotte 17 en sorte d'obtenir la pose à la fois de la bande de renforcement 10 et des deux rubans intercalaires séparables 19.

Lors de la mise en oeuvre du procédé précité, les deux rubans intercalaires 19 ont exactement la même largeur de coupe e que celle de la bande de renforcement 10.

La présente invention n'est pas limitée aux deux modes de réalisation qui ont été décrits à titre d'exemple non exhaustifs. En particulier, dans le second mode de réalisation, la bande d'attache 18 pourrait être fixée non pas sur le rabat 8 mais sur une patte d'attache fixée selon le bord 8a dudit rabat 8.

## Revendications

1. Article d'hygiène jetable du type couche-culotte, qui comporte une feuille extérieure imperméable (2), une feuille intérieure perméable (3), un matelas absorbant (4) pris en sandwich entre les deux dites feuilles (2,3), ledit article (1) étant configuré avec une zone d'entrejambe (7), une partie avant (5) et une partie arrière (6) et comportant des moyens d'attaches fixés latéralement à la partie arrière (6) et aptes à réaliser la solidarisation au moins temporaire des parties arrière (6) et avant (5) après positionnement de l'article (1) sur l'utilisateur par application sur une ou des bandes de renforcement (10) fixées sur la partie avant (5) de la feuille externe imperméable (2), **caractérisé en ce que** chaque moyen d'attache comporte des premiers éléments d'attache mécanique (14), **en ce que** l'article (1) est équipé d'un ruban intercalaire (13) séparable comportant sur une face (13b) une enduction adhésive (16) apte à solidariser ledit ruban intercalaire (13) et ladite bande de renforcement (10) pendant l'usage et sur l'autre face (13a) des seconds éléments d'attache mécanique (15), aptes à coopérer avec lesdits premiers éléments (14) pendant l'usage, et **en ce que** le ruban intercalaire (13) comporte une extrémité préhensile (13a) permettant son retrait de ladite bande de renforcement (10) et l'application de sa face (13b) adhésive sur la feuille extérieure (2) après usage.

2. Article selon la revendication 1 **caractérisé en ce que** chaque moyen d'attache consiste en une patte d'attache (9) dont une extrémité (12) est fixée latéralement sur un bord de la partie arrière (6) et qui comporte sur une face (11a) les premiers éléments d'attache mécanique (14).

3. Article selon la revendication 2 **caractérisé en ce que** la face interne du bord de la partie arrière (6) comporte une zone à adhérence limitée et **en ce que**, avant utilisation, la patte latérale d'attache (9) étant repliée, le ruban séparable (13) est disposé entre ladite zone à adhérence limitée et la patte d'attache (9) en sorte que sa face adhésive soit en contact avec la zone à adhérence limitée et que sa face pourvue des seconds éléments mécaniques soit en contact avec les premiers éléments mécaniques de la patte d'attache.

4. Article selon la revendication 3 **caractérisé en ce que** chaque patte d'attache (9) comporte une partie fixe (12) réalisant la fixation de ladite patte d'attache sur le bord de la partie arrière (6), et **en ce que** la zone à adhérence limitée est la face de ladite partie fixe (12) sur laquelle est repliée la patte d'attache (9) avant utilisation.

5. Article selon la revendication 1 **caractérisé en ce que** chaque moyen d'attache consiste en une bande (18) qui est pourvue des premiers éléments d'attache mécaniques et qui est fixée selon un bord (8a) de la face interne de la partie arrière (6).

6. Article selon la revendication 5 **caractérisé en ce que**, avant utilisation, la face adhésive du ruban intercalaire séparable (19) est appliquée sur la bande de renforcement (10).

7. Article selon l'une des revendications 5 ou 6 **caractérisé en ce que** le bord selon lequel est fixée la bande d'attache mécanique est rigidifié, par exemple par un élément de rigidification disposé sur la face extérieure de la partie arrière.

8. Article selon l'une des revendications 1 à 7 **caractérisé en ce que** le ruban intercalaire (13) séparable est un ruban textile ou non-tissé, présentant une surface en boucles ou fibreuse, et l'autre face revêtue d'une enduction adhésive.

9. Procédé de fabrication d'un article d'hygiène selon la revendication 5 avec une bande de renforcement équipée d'au moins un ruban intercalaire **caractérisé en ce que** :
a. on déroule en continu une bande complexe (20) composée de la superposition d'une première bande (21) en matériau plastique ayant une face adhésive (21a) et une face opposée (21b) et une seconde bande (22) ayant une face adhésive (22a) et une face (22b) pourvue d'éléments d'attache mécanique, la face adhésive (22a) de la seconde bande (22) étant appliquée sur la face opposée (21b) de la première bande (21),
b. on découpe transversalement des tronçons (23) de ladite bande complexe (20), et
c. on fixe chaque tronçon (23) sur la face extérieure de la partie avant (5) d'un article d'hygiène du type couche-culotte, en sorte d'obtenir la pose à la fois de la bande de renforcement (10) et du ruban intercalaire séparable (19).

10. Procédé selon la revendication 9 **caractérisé en ce que** s'agissant d'une bande de renforcement (10) unique s'étendant transversalement sur la largeur de la partie avant, la bande complexe (20) comprend deux secondes bandes (22) séparées l'une de l'autre, chacune d'un côté de la première bande (21).

## Patentansprüche

1. Wegwerfbarer-Hygieneartikel vom Slipeinlage-Typ, der eine undurchlässige äußere Lage (Folie) (2), eine durchlässige innere Lage (Folie) (3) und eine sandwichartig zwischen den beiden genannten Lagen (2, 3) angeordnete absorptionsfähige Polsterschicht (4) umfasst, wobei der genannte Artikel (1) besteht aus einer Zwickelzone (7), einem Vorderteil (5) und einem Hinterteil (6) und Befestigungs-Einrichtungen aufweist, die seitlich an dem Hinterteil (6) fixiert und geeignet sind, mindestens vorübergehend das Hinterteil (6) mit dem Vorderteil (5) zu verbinden, nachdem der Artikel (1) an dem Verbraucher positioniert worden ist durch Aufbringen der undurchlässigen äußeren Lage (2) auf ein oder mehrere Verstärkungsbänder (10), die auf dem Vorderteil (5) fixiert sind, **dadurch gekennzeichnet, dass** jede Befestigungseinrichtung erste mechanische Befestigungselemente (14) aufweist, dass der Artikel (1) mit einem abziehbaren Zwischenstreifen (13) ausgestattet ist, der auf einer Seite (13b) einen klebrigen Überzug (16) aufweist, um den genannten Zwischenstreifen (13) und das genannte Verstärkungsband (10) während des Gebrauchs miteinander zu verbinden, und auf der anderen Seite (13a) zweite mechanische Befestigungselemente (15) aufweist, die während des Gebrauchs mit den genannten ersten Elementen (14) kooperieren können, und dass der Zwischenstreifen (13) ein Griffende (13a) aufweist, so dass er nach dem Gebrauch von dem genannten Verstärkungsband (10) abgezogen werden kann und seine klebrige Seite (13b) auf die äußere Lage (2) aufgebracht werden kann.

2. Artikel nach Anspruch 1, **dadurch gekennzeichnet, dass** jede Befestigungseinrichtung aus einer Befestigungslasche (9) besteht, von der ein Ende (12) auf einem Rand des Hinterteils (6) seitlich fixiert ist und auf einer Seite (11a) die ersten mechanischen Befestigungselemente (14) aufweist.

3. Artikel nach Anspruch 2, **dadurch gekennzeichnet, dass** die Innenseite des Randes des Hinterteils (6) eine Zone mit begrenzter Haftung aufweist und dass vor dem Gebrauch, während die seitliche Befestigungslasche (9) umgebogen ist, der Abziehstreifen (13) zwischen der genannten Zone mit begrenzter Haftung und der Befestigungslasche (9) in der Weise angeordnet ist, dass seine klebrige Seite mit der Zone mit begrenzter Haftung in Kontakt steht und dass seine Seite, die mit zweiten mechanischen Befestigungselementen ausgestattet ist, mit den ersten mechanischen Befestigungselementen der Befestigungslasche in Kontakt steht.

4. Artikel nach Anspruch 3, **dadurch gekennzeichnet, dass** jede Befestigungslasche (9) einen festen Abschnitt (12) aufweist, der die genannte Befestigungslasche auf dem Rand des Hinterteils (6) fixiert, und dass die Zone mit begrenzter Haftung die Seite des genannten festen Abschnitts (12) ist, auf die die Befestigungslasche (9) vor dem Gebrauch umgefaltet ist.

5. Artikel nach Anspruch 1, **dadurch gekennzeichnet, dass** jede Befestigungseinrichtung aus einem Band (18) besteht, das mit ersten mechanischen Befestigungselementen ausgestattet ist und das auf einem Rand (8a) der Innenseite des Hinterteils (6) fixiert ist.

6. Artikel nach Anspruch 5, **dadurch gekennzeichnet, dass** vor dem Gebrauch die klebrige Seite des abziehbaren Zwischenstreifens (19) auf das Verstärkungsband (10) aufgebracht ist.

7. Artikel nach einem der Ansprüche 5 oder 6, **dadurch gekennzeichnet, dass** der Rand, auf dem das mechanische Befestigungsband fixiert ist, versteift ist, beispielsweise durch ein Versteifungselement, das auf der Außenseite des Hinterteils angeordnet ist.

8. Artikel nach einem der Ansprüche 1 bis 7, **dadurch gekennzeichnet, dass** der abziehbare Zwischenstreifen (13) ein Stoff- oder Vliesstoff-Streifen ist, der eine Schlingen- oder Faser-Oberfläche und eine mit einem klebrigen Überzug versehene andere Seite aufweist.

9. Verfahren zur Herstellung eines Hygieneartikels nach Anspruch 5 mit einem Verstärkungsband, das mit mindestens einem Zwischenstreifen ausgestattet ist, **dadurch gekennzeichnet, dass** man:
a) ein mehrteiliges Band (20) kontinuierlich abrollt, das, übereinanderliegend, besteht aus einem ersten Band (21) aus einem Kunststoffmaterial, das eine klebrige Seite (21a) und eine gegenüberliegende Seite (21b) aufweist, und einem zweiten Band (22), das eine klebrige Seite (22a) und eine mit mechanischen Befestigungselementen ausgestattete Seite (22b) aufweist, wobei die klebrige Seite (22a) des zweiten Bandes (22) auf die gegenüberliegende Seite (21b) des ersten Bandes (21) aufgebracht ist,
b) Endabschnitte (23) des genannten mehrteiligen Bandes (20) transversal abschneidet und
c) jeden Endabschnitt (23) auf der Außenseite des Vorderteils (5) eines Hygieneartikels vom Slipeinlage-Typ so fixiert, dass er gleichzeitig auf dem Verstärkungsband (10) und dem abziehbaren Zwischenstreifen (19) aufliegt.

10. Verfahren nach Anspruch 9, **dadurch gekennzeichnet, dass** im Falle eines einzigen Verstärkungsbandes (10), das sich quer über die Breite des Vorderteils erstreckt, das mehrteilige Band (20) zwei zweite Bänder (22) umfasst, die beiderseits des ersten Bandes (21) getrennt voneinander angeordnet sind.

## Claims

1. Disposable hygiene article of the nappy type, which comprises an impermeable outer sheet (2), a permeable inner sheet (3), an absorbent padding (4) sandwiched between the two said sheets (2,3), said article being configured with a crotch area (7), a front portion (5) and a rear portion (6) and comprising fastening means fixed laterally to the rear portion (6) and capable of realising the at least temporary interlocking of the rear (6) and front (5) portions after positioning of the article (1) on the user by application of one or more fixed reinforcement strips (10) on the front portion (5) of the outer impermeable sheet (2), **characterised in that** each fastening means comprises first mechanical fastening elements (14), **in that** the article (1) is provided with a separable inserted ribbon (13) comprising on one face (13b) an adhesive coating (16) capable of interlocking said inserted ribbon (13) and said reinforcement strip (10) during use, and on the other face (13a) second mechanical fastening elements (15) capable of co-operating with said first elements (14) during use, and **in that** the inserted ribbon (13) comprises a gripping end (13a) permitting its withdrawal from said reinforcement strip (10) and the application of its adhesive face (13b) to the outer sheet (2) after use.

2. Article according to claim 1, **characterised in that** each fastening means consists of a fastening tab (9), one end (12) of which is fixed laterally on an edge of the rear portion (6) and which comprises on one face (11a) the first mechanical fastening elements (14).

3. Article according to claim 2, **characterised in that** the inner face of the edge of the rear portion (6) comprises an area with limited adhesion, and **in that**, before use, the lateral fastening tab (9) being folded back, the separable ribbon (13) is disposed between said area with limited adhesion and the fastening tab (9) so that its adhesive face is in contact with the area with limited adhesion and so that its face provided with the second mechanical elements is in contact with the first mechanical elements of the fastening tab.

4. Article according to claim 3, **characterised in that** each fastening tab (9) comprises a fixed portion (12) realising the fixing of said fastening tab on the edge of the rear portion (6), and **in that** the area with limited adhesion is the face of the fixed portion (12) on which the fastening tab (9) is folded back before use.

5. Article according to claim 1, **characterised in that** each fastening means consists of a tape (18) which is provided with first mechanical fastening elements and which is fixed along an edge (8a) of the inner face of the rear portion (6).

6. Article according to claim 5, **characterised in that**, before use, the adhesive face of the separable inserted ribbon (19) is applied to the reinforcement strip (10).

7. Article according to one of claims 5 or 6, **characterised in that** the edge along which the mechanical fastening tape is fixed is stiffened, for example by a stiffening element disposed on the outer face of the rear portion.

8. Article according to one of claims 1 to 7, **characterised in that** the separable inserted ribbon (13) is a textile or non-woven ribbon having a looped or fibrous surface and the other face being covered with an adhesive coating.

9. Method of manufacturing a hygiene article according to claim 5, with a reinforcement strip provided with at least one inserted ribbon, **characterised in that**:
a. a complex tape (20) is continuously unwound, composed of the superimposition of a first tape (21) formed from a plastics material and having an adhesive face (21a) and an opposite face (21b), and a second tape (22) having an adhesive face (22a) and a face (22b) provided with mechanical fastening elements, the adhesive face (22a) of the second tape (22) being applied to the opposite face (21b) of the first tape (21),
b. sections (23) of said complex tape (20) are cut transversely, and
c. each section (23) is fixed on the outer face of the front portion (5) of a hygiene article of the disposable nappy type so as to obtain the simultaneous fitting of the reinforcement strip (10) and of the separable inserted ribbon (19).

10. Method according to claim 9, **characterised in that,** since there is a sole reinforcement strip (10) extending transversely over the width of the front portion, the complex tape (20) comprises two second tapes (22) separated the one from the other, each by one side of the first tape (21).
